# EUROPEAN PATENT APPLICATION

(11) **EP 2 343 554 A1**
(43) Date of publication of application: **13.07.2011**
(21) Application number: 09816300.9
(22) Date of filing: 24.09.2009
(51) Int. Cl.: G01N 33/68, A01K 67/027, C12Q 1/06, C12Q 1/68, G01N 33/53, G01N 33/543, C12N 15/09

(54) **BIOMARKER FOR MICRODOMAIN DISORDER**

(30) Priority: 25.09.2008 JP 2008246846
(71) Applicant: Sumitomo Chemical Company, Limited, Tokyo 104-8260 (JP)
(72) Inventor: HIGASHI, Kiyoshi, Osaka-shi Osaka 542-0012 (JP); MIKAMI, Toshiyuki, Takarazuka-shi Hyogo 665-0804 (JP)
(74) Representative: Vossius & Partner
(86) International application number: PCT/JP2009/067130
(87) International publication number: WO 2010/035888

(57) **Abstract**

The present invention relates to a diagnostic technique related to a biomarker for a microdomain disease and a method for detecting a microdomain disease of which manipulation is easy and which is inexpensive.

## Description

### TECHNICAL FIELD

The present invention relates to a biomarker for a microdomain disease, and so on.

### BACKGROUND ART

Obesity, which is one of the causes of lifestyle-related diseases, is a state in which the number of adipocytes has increased and an adipocyte itself has been hypertrophic, and generally thought to be a trigger which causes onset of pathology of diabetes mellitus, hypertension, arteriosclerosis, stroke, myocardial infarction, and so on. Bioactive substances produced by an adipocyte are collectively called adipocytokines. These substances originally play an important role in metabolism of an adipocyte itself. However, when abnormality in secretion control such as excess secretion or hyposecretion is lead by obesity or the like, the substances become a cause which triggers onset of pathology. For example, as for plasminogen activator inhibitor 1 (PAI-1), which is an important regulatory factor in fibrinolytic system, it is thought that, when fat accumulation occurs, level of expression thereof remarkably increases especially in visceral fat and blood level also increases, thereby becoming one of the causes of a vascular complication. In addition, it is thought that resistin, of which molecular weight is about 20 kDa, suppresses a stimulatory action by insulin on sugar uptake, thereby being a factor involved in onset of type 2 diabetes mellitus as with TNF-α or the like. It is thought that the suppressive mechanism thereof inhibits signaling of insulin by activating a signaling molecule such as SOCS3 or NF-kB, in other words, induces insulin resistance.

Furthermore, in an obese patient, although leptin, which suppresses eating, is present in blood in a high concentration, leptin resistance to attenuate an action of leptin is observed. Therefore, even if leptin secreted from an adipocyte acts on the hypothalamus, eating behavior is not suppressed, whereby a hyperphagia state remains.

Moreover, recently, relevance between Alzheimer's disease, which is a representative disease of dementia, and life style-related diseases has been attracting attention. For example, it is known that prevalence of Alzheimer's disease among diabetic patients is twice the prevalence among healthy persons. Nerve Growth Factor (NGF) is a protein which enhances proliferation of a neuronal cell and suppresses cell death of a neuronal cell. It is thought that a diabetic patient is likely to be affected with Alzheimer' s disease since the action of NGF is attenuated in a diabetic patient.

There has been proposed a hypothesis that pathology of lifestyle-related diseases such as obesity, diabetes mellitus and a complication thereof is an illness wherein the composition, structure and function of a microdomain of a plasma membrane is changed by abnormal expression of a glycosphingolipid, whereby signaling by a cytokine or a hormone becomes abnormal, in other words, a microdomain disease. Inokuchi, et al. have found that TNF-α, produced by an adipocyte or an inflammatory cell enhances expression of ganglioside GM3 synthetase present in a cell via a TNF-α receptor and GM3, which is a component of a microdomain, increases, whereby the structure of the microdomain changes, to attenuate the function of an insulin receptor present in the microdomain (Proc. Natl. Acad. Sci. USA (2007), 104, p. 13678-13683). However, it has not ever been reported that TNF-α induces leptin resistance or suppression of the action of NGF via GM3.

Until now, as a method to make a diagnosis of insulin resistance, a glucose tolerance test has been generally used, but there is a problem in terms of ease. In addition, a biomarker which can make a diagnosis of leptin resistance or suppression of an action of NGF has not ever been known. Therefore, a technique related to a biomarker for a microdomain disease or a method for detecting a microdomain disease of which manipulation is easy and which is inexpensive is anticipated.

### DISCLOSURE OF INVENTION

An object of the present invention is to provide a technique related to a biomarker for a microdomain disease and a method for detecting a microdomain disease of which manipulation is easy and which is inexpensive.

The present invention focuses on an adipocytokine secreted to the outside of a cell in association with hypertrophy of an adipocyte, and discloses that the amount of GM2A (Ganglioside GM2 Activator) in an adipose tissue increases in a diabetes mellitus model animal, a diabetic patient and an obese human as compared with that in a normal animal or a healthy person and that GM2A suppresses signaling of insulin, leptin and a cytokine such as NGF.

That is, the present invention is intended for use of GM2A as a biomarker for a microdomain disease.

More specifically, the present invention provides:
[1] a method for detecting a microdomain disease in a test animal comprising a step of measuring the amount or activity of GM2A in a biological sample obtained from the test animal and a step of comparing the amount or activity of GM2A with a threshold;
[2] a method for monitoring change in a disease state of amicrodomain disease comprising a step of measuring the amount or activity of GM2A in a biological sample obtained from a test animal and a step of comparing the amount or activity of GM2Awith the amount or activity of GM2A measured for the test animal at differing time points;
[3] the method according to the above [1] or [2], wherein the biological sample is blood, lymph, a tissue or a cell;
[4] the method according to the above [1] or [2], wherein the measurement of the amount or activity of GM2A is carried out by enzyme immunoassay, radioimmunoassay, fluoroimmunoassay, ELISA, immunohistochemical staining, immunoprecipitation, Western blotting, Northern blotting or RT-PCR;
[5] use of GM2A as a biomarker for a microdomain disease;
[6] the use according to the above [5], wherein the microdomain disease is obesity, hyperlipidemia, hypertension, arteriosclerosis, diabetes mellitus or a complication thereof, cancer, central nervous system disorder, endometriosis, osterioporosis or autoimmune disease;
[7] a kit for diagnosis of a microdomain disease which comprises a reagent for measuring the amount or activity of GM2A;
[8] the kit for diagnosis according to the above [7], wherein the reagent for measuring the amount or activity of GM2A is an anti-GM2A antibody, or a ganglioside labeled with a radioisotope or a fluorescent dye;
[9] a transgenic animal into which a gene modified so that the expression level of GM2A can increase has been introduced;
[10] the transgenic animal according to the above [9], wherein the expression level of GM2A is increased in an adipocyte;
[11] an animal to which GM2A protein is administered to artificially increase the amount or activity of GM2A in blood;
[12] the animal according to any one of the above [9] to [11], wherein a symptom of a microdomain disease is exhibited;
[13] a prophylactic or therapeutic agent for a microdomain disease which comprises an anti-GM2A antibody as an active ingredient; and
[14] a prophylactic or therapeutic agent for a microdomain disease which comprises a GM2A-inhibitory active compound as an active ingredient; and so on.

According to the present invention, a technique related to a biomarker for a microdomain disease and a method for detecting a microdomain disease of which manipulation is easy and which is inexpensive is provided.

### MODE FOR CARRYING OUT THE INVENTION

One aspect of the present invention is use of GM2A as a biomarker for a microdomain disease. More specifically, GM2A is used as a biomarker for a microdomain disease as follows.

### [1] Method for Detecting Microdomain Disease

The method for detecting a microdomain disease of the present invention includes a step of measuring the amount or activity of GM2A in a biological sample obtained from a test animal and a step of comparing the measured amount or activity of GM2A with a threshold.

In the present specification, the term "microdomain disease" means an illness wherein the composition, structure and function of a microdomain of a plasma membrane (for example, a raft such as a caveola) has been changed by abnormal expression of glycosphingolipid and signaling by a cytokine or a hormone such as insulin, leptin or nerve growth factor (NGF) is abnormal.

Specific examples of a "microdomain disease" include obesity, hyperlipidemia, hypertension, arteriosclerosis, diabetes mellitus and complications thereof (for example, diabetic retinopathy, diabetic nephropathy, and diabetic neuropathy), cancer, central nervous system disorder (for example, Alzheimer's disease), endometriosis, osterioporosis, and autoimmune disease. Among them, the present invention can be suitably applied to obesity, hyperlipidemia, hypertension, arteriosclerosis, diabetesmellitus and complications thereof (for example, diabetic retinopathy, diabetic nephropathy, and diabetic neuropathy), or central nervous system disorder (for example, Alzheimer's disease). In the present invention, obesity preferably results from leptin resistance, and central nervous system disorder (for example, Alzheimer's disease) preferably results from suppression of NGF action.

In the present specification, "detecting a microdomain disease" is used with the intention to encompass judging whether a test animal is affected with a microdomain disease or not. In addition, in the present specification, "affected with a microdomain disease" is used with the intention to encompass the case in which a surface symptom of a microdomain disease is not observed.

A test animal in the present invention is preferably a nonhuman animal or a human, more preferably a nonhuman mammal or a human, and especially preferably a human.

A biological sample in the present invention is preferably blood, lymph, a tissue (for example, an adipose tissue) or a cell (for example, an adipocyte).

GM2A is a glycoprotein involved in metabolism of a glycosphingolipid. A function thereof is to serve as a coenzyme involved in degradation of a ganglioside, for example, to cooperate with saposin B to degrade GM1 into GM2, or otherwise to cooperate with hexosaminidase A to degrade GM2 into GM3 and GA2 into lactosylceramide. GM2A precursor of which amino-terminal peptide has been removed is secreted to the outside of a cell. For example, human GM2A is a publicly known glycoprotein of which molecular weight is about 25 kDa, and which is composed of 199 amino acid residues, and is secreted to the outside of a cell after its amino-terminal 31 residues are cleaved.

In the present invention, GM2A to be measured may be any GM2A that may naturally occur, and may be, for example, full-length GM2A (GM2Aprecursor) of GM2Aor those of which amino-terminal polypeptide has been removed and secreted to the outside of a cell.

A method for measuring the amount of GM2A is not limited so long as it is a method which can determine the amount of GM2A, and, for example, used are a method for measuring the level of expression of GM2A at the translation level such as enzyme immunoassay (EIA), radioimmunoassay (RIA), fluoroimmunoassay (FIA), ELISA, immunohistochemical staining, high performance liquid chromatography, mass spectrum determination method, enzyme method, immunoprecipitation, or Western blotting, a method for measuring the level of expression of GM2A at the glycosylation level such as lectin histochemical staining or lectin blotting, and a method for measuring the level of expression of GM2A at the transcription level such as Northern blotting, RT-PCR or in situ hybridization, using a specific antibody to GM2A protein, lectin or a specific antibody to a sugar chain of GM2A, or a nucleic acid which binds to a GM2A gene.

In addition, a method for measuring the activity of GM2A is not limited so long as it is a method which can measure the activity of GM2A, and, for example, used is a method for determining the quantity of a product or a bound substance using a ganglioside labeled with a radioisotope or a fluorescent dye as a substrate in the concurrent presence or absence of saposin, hexosaminidase, or the like.

By using these methods, it is capable of quantitatively measuring the amount or activity of GM2A. In addition, by using an autoanalyzer or the like, it is capable of measuring a lot of biological samples in a short time.

An antibody used in an immunological measurement method such as enzyme immunoassay (EIA) is not specifically limited, and, for example, a polyclonal antibody or a monoclonal antibody is used, and preferably a monoclonal antibody is used. In addition, in GM2A, the position of an epitope recognized by an antibody is not specifically limited. An antibody may be a entire antibody molecule, or may be an antibody fragment which can specifically bind to an antigen such as a Fab fragment or a F(ab')₂ fragment.

The present invention is to measure the amount or activity of GM2A in a biological sample obtained from a test animal by the method as described above and to judge that the test animal is affected with a microdomain disease when the obtained measured value is higher than the threshold preset with reference to the amount or activity of GM2A in a biological sample of a healthy subject and an animal affected with a microdomain disease.

Here, the healthy subject means a test animal not affected with a microdomain disease, and can encompass a test animal affected with other diseases.

Since the threshold varies according to the species, sex, age (age in weeks) and lifestyle of the test animal, it is necessary to preset the threshold for each of the factors.

The threshold can be set, for example, by measuring the amount or activity of GM2A in a biological sample of a healthy subject and multiple animals affected with a microdomain disease and referring to the average value ± standard deviation of the measured value obtained for each subject. As a method to judge whether a subj ect is a healthy subj ect or an animal affected with a microdomain disease for threshold decision, a publicly known method for diagnosing an illness encompassed by a microdomain disease can be utilized. Specific examples of setting of such a threshold include (the average value - standard deviation of the amount or activity of GM2A in a biological sample of an animal affected with a microdomain disease).

Here, the "average value" is an arithmetic average obtained by measuring the amount or activity of GM2A of each of biological samples obtained from multiple solids which are animals of an identical species and dividing the sum of these measured values by the number of individuals (number of samples).

The threshold set as described above is compared with the amount or activity of GM2A in a biological sample obtained from a test animal, and the test animal is judged to be affected with a microdomain disease when the measured value is equal to or higher than the threshold.

Especially, it is expected that the method of the present invention can detect a microdomain disease early, for example, in the case of diabetes mellitus, earlier than the time when diabetes mellitus is detected by a blood glucose test.

### [2] Method for Monitoring Change in Disease State of Microdomain Disease

The method for monitoring change in a disease state of a microdomain disease of the present invention includes a step of measuring the amount or activity of GM2A in a biological sample obtained from a test animal and a step of comparing the measured amount or activity of GM2Awith the amount or activity of GM2Ameasured for the test animal at differing time points.

The method can be conducted basically in the same manner as the above-mentioned "method for detecting a microdomain disease" of the present invention, but the method is different in that the amount or activity of GM2A is measured for one test animal at differing time points and the measured amounts or activities of GM2A are compared with each other.

The amount or activity of GM2A is measured at a certain interval (for example, 1 day, 3 days, 1 week, 2 weeks, or 1 month).

When the obtained measured value increases or decreases over time, it can be judged that a microdomain disease is exacerbated or improved. By carrying out such monitoring, selection of an appropriate therapeutic agent or a prophylactic agent and determination of the effect thereof can be effectively carried out in the animal judged to be affected with or likely to be affected with a microdomain disease.

It can be utilized for decision of an appropriate treatment plan and evaluation of a new drug.

### [3] Kit for Diagnosis of Microdomain Disease

The present invention also provides a kit for diagnosis of a microdomain disease, which can be used for conducting the method of the present invention explained above. This kit includes a reagent for measuring the amount or activity of GM2A. As such a measuring reagent, a reagent which can recognize GM2A such as an anti-GM2A antibody, or a reagent which is to be degraded by cooperation of GM2A and saposin B or lacthexosaminidase A such as a ganglioside labeled with a radioisotope or a fluorescent dye can be used.

An anti-GM2A antibody, being not specifically limited, is preferably a monoclonal antibody. When the amount of GM2A is measured by an immunological technique, the kit according to the present invention may further include a substance and an instrument or the like which can be used for immobilization of an antibody, detection of an antibody or the like. For immobilization of an antibody, a carrier such as a microtiter plate, a liquid for immobilization such as a carbonate buffer, a blocking solution containing gelatin, albumin or the like can be included. For detection of an antibody, it is preferable to label the antibody in advance, in which case the kit according to the present invention can include a reagent for the detection. For example, when biotin is used as a labeling substance, a conjugate of streptavidin and horseradish peroxidase (HRP), and a color solution which develops color by the action of HRP can be included as a reagent for detection. In addition, a substrate used for measuring the activity of GM2A is preferably a ganglioside labeled with a radioisotope or a fluorescent dye.

### [Animal]

The present invention also provides a transgenic animal into which a gene modified so that the expression level of GM2A can increase has been introduced. These transgenic animals can be easily prepared by one skilled in the art using a gene transfer technique commonly used in this technical field.

A gene modified so that the expression level of GM2A can increase is a gene comprising a DNA fragment in which an enhancer sequence portion, a promoter sequence portion and a poly A signal sequence portion of a given gene described below are linked to a GM2A gene.

The tissue or cell in which a GM2A gene is expressed can be determined according to the given gene to which a GM2A gene is linked.

Regarding the given gene, for example, a GM2A gene can be highly expressed in an adipocyte when an aP2 gene expressed only in an adipocyte is used, and a GM2A gene can be highly expressed in a neuronal cell when a neuron-specific enolase gene expressed in a neuronal cell is used. Examples of other given genes include an albumin gene and an insulin gene. A transgenic animal into which a gene modified so that the expression level of GM2A can increase has been introduced is preferably a transgenic animal of which expression level of GM2A is increased in an adipocyte such as a transgenic animal in which a GM2A gene is highly expressed in an adipocyte as described above.

Examples of the enhancer sequence portion include virus enhancers such as SV40 enhancer andpolyomavirus enhancer, enhancers of a gene of the immune system such as IgH enhancer, IgL enhancer and T-cell receptor α chain enhancer, and cell enhancers such as β-actin enhancer, MCK enhancer and elastase I gene enhancer.

Examples of the promoter sequence portion include promoter sequence portions derived from viruses (for example, cytomegalovirus, Moloney leukemia virus, JC virus, and mammary tumor virus), and promoter sequence portions of metallothionein, metalloproteinase 1 tissue inhibitor, α-smooth muscle actin, polypeptide chain elongation factor 1α, β-actin, α- and β-myosin heavy chain, myosin light chain 1 and 2, and myelin basic protein.

Examples of the poly A signal sequence portion include SV40 poly A signal and growth hormone-poly A signal.

Preparation of a transgenic animal into which a gene modified so that the expression level of GM2A can increase has been introduced can be conducted by introducing a gene comprising the DNA fragment into a fertilized egg of a nonhuman mammal by microinjection or the like, transplanting the fertilized egg into a pseudopregnant female nonhuman mammal, and delivering the nonhuman mammal. As a nonhuman mammal, for example, in addition to rodents such as a mouse, a hamster, a guinea pig, a rat and a rabbit, a chicken, a dog, a cat, a goat, a sheep, cattle, a pig, a monkey or the like can be used. Rodents such as a mouse, a hamster, a guinea pig, a rat and a rabbit are preferable from the viewpoint of ease in preparation, fostering and use and among them, a mouse is most preferable (Endocrine Journal (2008), 55(4), p. 767-776).

The present invention also provides an animal to which GM2A protein has been administered and of which amount or activity of GM2A in blood is artificially increased.

Examples of the method to administer GM2A protein to an animal include intravenous administration, intraperitoneal administration, intradermal administration, subcutaneous administration, aerosol administration, and intraventricular administration. In addition, among the methods to administer GM2A protein to an animal, examples of a method for administration to an animal from inside of the body further include a method to implant GM2A protein under or into the skin or the like in the form of a pellet, and a method to seal GM2A protein in an osmotic pump and implant the GM2A protein under or into the skin or into the peritoneal cavity or the like.

The animal is not specifically limited, and preferably a nonhuman animal, more preferably a nonhuman mammal, being exemplified by a mouse, a rat, a hamster, a guinea pig, a rabbit, a dog, a cat, a horse, cattle, a sheep, a pig, a goat and a monkey.

As described above, an animal in which expression of GM2A gene in the blood or tissue or the amount or activity of GM2A is increased is very useful as a model animal for drug development such as a prophylactic or therapeutic agent for a microdomain disease or as an animal for screening of the pharmaceutical, since the animal can exhibit symptoms of a microdomain disease.

### [Transformed Cell]

The present invention also provides an animal cell into which a gene modified so that the expression level of GM2A can increase has been introduced. Such a transformed cell can be easily prepared by one skilled in the art using a gene transfer technique commonly used in this technical field.

For example, a transformed animal cell in which expression of GM2A gene is increased can be prepared by linking an enhancer and a promoter of a gene expressed only in an adipocyte or those modified for high expression thereof to a GM2A gene to be introduced and introducing the gene into a chromosome. The GM2A gene to be introduced is not specifically limited so long as the gene is translated to exhibit the function of GM2A, and may be a modified gene.

A host cell is not specifically limited, and an animal cell such as a murine myeloma cell, a Chinese hamster ovary (CHO) cell, a COS-7 cell, a Vero cell, a HeLa cell, or a GHS cell derived from a rat is used.

Such a transformed animal cell can be used for drug development of a prophylactic or therapeutic agent or the like for a microdomain disease, and so on.

### EXAMPLES

The present invention will be explained more specifically by way of the following examples.

### Example 1 (Quantitative Determination of GM2A Gene in Normal Rat Adipocyte)

A normal rat adipocyte (a visceral adipocyte, a subcutaneous adipocyte, or a epididymal adipocyte) and a liquid medium (adipocyte differentiation medium) were purchased from Primary Cell Co. , Ltd. GM2A primers (shown by SEQ ID NOs: 1 and 2 below) were obtained by consigning the synthesis thereof to Invitrogen.

To each adipocyte (0.75 × 10⁶ cells), 6 ml of a liquid medium was added to disperse the cells, and the obtained adipocyte dispersion was centrifuged at 500 rpm for 5 minutes. After adding 3.2 ml of a liquid medium to the precipitate to disperse the cells again, the obtained adipocyte redispersion was added to a 24 well plate at the rate of 0.5 ml per well. The cells were cultured at 37°C under an atmosphere of 5% CO₂. On the following day (referred to as Day 1), to the obtained culture was added 0.5 ml of a liquid medium and the culture was cultured overnight. Thereafter, the liquid medium was totally exchanged every 2 days. The adipocyte was washed with PBS(-) on each of Days 2, 4, 6 and 10, and total RNA was prepared from the washed adipocyte using RNeasy Mini Kit (catalogue No. 74106, Qiagen).

Next, 12.5 µl of 10 ng/µl total RNA, 2. 5 µl of 20 µM oligo (dT), 2.5 µl of 10 mM dNTP and 12.5 µl of purified water (30 µl in total) were mixed, and the obtained mixture was kept at 65°C for 5 minutes, and then cooled in ice. To the cooled mixture were added 10 µl of a 5 x buffer, 5 µl of 0.1 M DTT, 1.25 µl of SuperScript III Reverse Transcriptase (catalogue No. 18080-044, Invitrogen), 1.25 µl of RNase OUT (catalogueNo. 10777-019, Invitrogen) and 2.5 µl of purified water (20 µl in total) and mixed, and the obtained mixture was reacted at 42°C for 60 minutes and at 99°C for 3 minutes. Thereafter, the mixture was kept at 4°C, whereby a cDNA solution was prepared.

Next, 5 µl of a 10 x PCR buffer, 5 µl of 2 mM dNTP, 2 µl of 25 mM MgSO₄, 1 µl of KOD-Plus- (catalogue No. KOD-201, TOYOBO CO., LTD.), 1 µl each of 10 µM GM2A primers (shown by SEQ ID NOs: 1 and 2 below), 2 µl of a cDNA solution and 33 µl of purified water (50 µl in total) were mixed, and the obtained mixture was subjected to PCR (the conditions were: 1 cycle: 94°C for 30 seconds; 29 cycles: 94°C for 15 seconds, 56°C for 30 seconds; and 68°C for 1 minute, and 1 cycle: 68°C for 7 minutes). In addition, instead of a GM2AP primer, a GAPDH primer (Applied Biosystems) was added, and similar manipulation (PCR) was carried out. Using a 1% agarose gel containing ethidium bromide, 5 µl of the produced PCR product was electrophoresed. Thereafter, the fluorescence image in the agarose gel after the electrophoresis was imported using Image Reader LAS-1000 (manufactured by FUJIFIUM Corporation), and the fluorescence intensity in the fluorescence image was measured using Image Gauge (manufactured by FUJIFIUM Corporation). Relative level of expression of a GM2A gene was calculated based on the following formula, and the results are shown in Table 1.

Relative level of expression of GM2A gene = fluorescence intensity of GM2A/fluorescence intensity of GAPDH

### [Rat GM2AP Primer]

SEQ ID NO: 1: gtgctgggct tgctgttc
SEQ ID NO: 2: gatgctctgg atgcggtagt

**Table 1**

| | Relative Day 2 | Expression Day 4 | Level of Day 6 | GM2A Gene Day 10 |
|---|---|---|---|---|
| Visceral Adipocyte | 0.01 | 0.12 | 0.26 | 0.39 |
| Subcutaneous Adipocyte | 0.19 | 0.71 | 0.78 | 0.88 |
| Epididymal Adipocyte | 0.04 | 0.38 | 0.43 | 0.33 |

As a result of microscopic observation, a lipid droplet in each adipocyte accounted for about 10% of cytoplasm on Day 2, about 30% of cytoplasm on Day 4, and about 90% on day 6. On the other hand, in all of the adipocytes, relative level of expression of GM2A gene clearly increased on Day 4 and later as compared with that on Day 2. That is, the relative level of expression of GM2A gene had already increased from the stage in which the amount of lipid droplet was small.

### Example 2 (Quantitative Determination of GM2A Gene in Each Tissue of Diabetes Mellitus Model Animal)

As a representative diabetes mellitus model animal, a 15-week-old KK-Ay mouse (CLEA Japan, Inc.) and a 16-week-old db/db mouse (CharlesriverLaboratories Japan Inc.) were used. In addition, as a normal animal, a 16-week-old C57BL mouse (Charlesriver Laboratories Japan Inc.) was used. First, after each mouse was exsanguinated to death, the epididymal adipose tissue, the femoral muscle, the liver, the kidney, the spleen, and the testis were collected, respectively. Next, after about 30 mg of each tissue was homogenized in 1 ml of RNA later (catalogue No. 7021, Ambion, Inc.) using Handy Pestle (catalogue No. HMX-301, TOYOBO CO., LTD.), the total RNA was prepared using RNeasy Mini Kit from a portion of the supernatant resulting after standing of the obtained homogenate.

Next, a cDNA solution was prepared from the prepared total RNA by a method similar to the method used in Example 1. Then, the level of expression of GM2A gene in each tissue was measured using TaqMan Gene Expression Assays (Assay ID: Mm00494656-ml, Applied Biosystems). That is, 1 µl of Assay solution, 10 µl of 2 x Fast Master Mix (catalogue No. 4304437, Applied Biosystems), 5 µl of a cDNA solution and 4 µl of purified water (20 µl in total) were mixed, and the obtained mixture was subjected to PCR using 7900HT Fast Real-Time PCR System (manufactured by Applied Biosystems). In addition, instead of the above-mentioned Assay solution, Rodent GAPDH primer and Rodent GAPDH probe (Applied Biosystems) were added and similar manipulation (PCR) was carried out, whereby the level of expression of GAPDH gene was measured. Here, as a standard solution, a solution obtained by diluting a cDNA solution prepared from a testis twofold for each was prepared and used.

The conditions of PCR were: 1 cycle: 94°C for 30 seconds; 40 cycles: 94°C for 15 seconds, 56°C for 30 seconds, and 68°C for 1 minute; and 1 cycle: 68°C for 7 minutes, and each gene dosage was measured on the basis of a standard curve. Here, relative level of expression of GM2A gene was calculated based on the following formula. The results are shown in Table 2.

Relative level of expression of GM2A gene = level of expression of GM2A/level of expression of GAPDH

**Table 2**

| | Relative Expression Level of GM2A Gene | | | | | |
|---|---|---|---|---|---|---|
| | Adipose Tissue | Femoral Muscle | Liver | Kidney | Spleen | Testis |
| C57BL Mouse | 0.08 | 0.01 | 0.12 | 0.13 | 0.27 | 1.22 |
| KK-Ay Mouse | 0.20 | 0.05 | 0.08 | 0.10 | 0.51 | 1.44 |
| db/db Mouse | 0.23 | 0.02 | 0.09 | 0.11 | 0.19 | 1.36 |

In the diabetes mellitus model mouse, the level of expression of GM2A gene in the adipose tissue clearly increased as compared with the C57BL mouse. On the other hand, in other tissues, no clear difference was observed between the diabetes mellitus model mouse and the normal mouse.

### Example 3 (Quantitative Determination of GM2A Protein in Adipose Tissue in Diabetes Mellitus Model Animal)

To about 30 mg of an epididymal adipose tissue of a diabetes mellitus model mouse and a normal mouse, 200 µl of an RIPA buffer (50 mM Tris-HCl (pH 8.0), 50 mM NaCl, 1% Nonidet P-40, 0.5% sodium deoxycholate, 0.1% SDS, 1 mM EDTA, 1 mM PMSF, 1 mM Na₃VO₄, 10 mM NaF, 15 µg/ml aprotinin, 10 µg/ml leupeptin) was added, and the obtained mixture was sonicated in ice. Next, the obtained homogenate was centrifuged at 15,000 rpm for 5 minutes at 4°C. The obtained supernatant was referred to as an adipose tissue extract. Here, protein concentration of each adipose tissue extract was measured using BCA Protein Assay Reagent (catalogue No. 23228, Pierce), using bovine serum albumin as a standard protein.

After subjecting 100 µg of the adipose tissue extract to 10-20% SDS-PAGE, each separated protein was blotted to a PVDF membrane (catalogue No. RPN303F, GE Healthcare). After the PVDF membrane was blocked with a TBS-T solution (10 mM Tris-HCl (pH 7.5), 0.15 M NaCl, 0.1% Tween-20) containing 5% skimmed milk (catalogue No. 198-10605, Wako Pure Chemical Industries, Ltd.), a rabbit anti-GM2A antibody (catalogue No.10864-2-AP, Proteintech Group Inc.) diluted 500-fold with a TBS-T solution was added thereto, and the membrane was left at room temperature for 1 hour. Next, after washing the obtained PVDF membrane with a TBS-T solution 3 times, HRP-labeled goat anti-rabbit IgG antibody (catalogue No. sc-2004, Santa Cruz Biotechnology, Inc.) diluted 1000-fold with a TBS-T solution was added thereto, and the membrane was left at room temperature for 1 hour. After washing the obtained PVDF membrane with a TBS-T solution 3 times, the membrane was immersed in Chemi-Lumi One (catalogue No. 05027-20, NACALAITESQUE, INC.), and the luminescence image was imported by Image Reader LAS-1000. The luminescence intensity corresponding to the band of GM2A protein was measured using Image Gauge. Next, WB Stripping Solution (catalogue No. 05364-55, NACALAI TESQUE, INC.) was added to the PVDF membrane, and kept at 37°C on shaking for 1 hour. Thereafter, the PVDF membrane was reacted with goat anti-actin antibody (catalogue No. sc-1616, Santa Cruz Biotechnology, Inc.) and HRP-labeled donkey anti-goat IgG antibody(catalogue No.sc-2020, Santa CruzBiotechnology, Inc.) diluted 1000-fold, and the luminescence intensity corresponding to the band of actin protein was measured as in the same manner as described above. The relative level of expression of GM2A protein was calculated based on the following formula. The results are shown in Table 3.

Relative level of expression of GM2A protein = amount of luminescence of GM2A protein/amount of luminescence of actin protein

**Table 3**

| | Relative Expression Level of GM2A Protein in Adipose Tissue |
|---|---|
| C57BL Mouse | 0.34 |
| KK-Ay Mouse | 0.72 |
| db/db Mouse | 0.43 |

In the diabetes mellitus model mouse, the level of expression of GM2A protein in an adipose tissue increased as compared with that in the C57BL mouse.

### Example 4 (Expression Analysis of GM2A Gene in Obese Patient)

Distribution of expression of GM2A gene in a human normal tissue was examined using Ascenta of Gene Logic Inc. , which is a commercially available database listing gene expression data by the tissue and the disease of a human. As a result of examining expression of 212737_at, which is a probe corresponding to GM2A gene, in every human tissue, expression of GM2A gene was observed in almost all of the tissues. Next, expression of GM2A gene in the adipose tissue, the skeletal muscle and the liver of an obese patient was compared with that of a healthy subject. The results are shown in Table 4.

**Table 4**

| | Relative Expression Level of GM2A Gene | | |
|---|---|---|---|
| | Adipose Tissue | Skeletal Muscle | Liver |
| Nonobese | 1.00 | 1.00 | 1.00 |
| Obese | 1.47*** | 0.98 | 1.04 |

| | | | |
|---|---|---|---|
| *** p < 0.005 | | | |

In the liver and the skeletal muscle, no significant difference in the level of expression of GM2A gene was observed between the obese patient and the nonobese patient. On the other hand, in the adipose tissue, significantly higher expression of GM2A gene was observed in obese patients relative to that in nonobese patients (p < 0.005).

### Example 5 (Inhibitory Action by GM2A Protein on Intracellular Signaling)

Mouse skeletal muscle cell line C2C12 (CRL-1772) and rat pheochromocytoma cell line PC12 (CR1-1721) were purchased fromATCC. Cells of the both cell lines were cultured in a D-MEMmedium containing 10% inactivated fetal bovine serum (catalogue No. 14247-15, NACALAI TESQUE, INC.) at 37°C under an atmosphere of 5% CO₂. Cells of differentiated mouse skeletal muscle cell line C2C12 were prepared by exchanging the medium for a D-MEM containing 2% inactivated equine serum and culturing the mixture for 8 to 10.

Human GM2A protein was prepared as follows. First, a cDNA was prepared from human lung cancer cell line HARA-B (catalogue No. JCRB1080.1, Health Science Research Resources Bank), using oligo(dT). Using the prepared cDNA, a PCR fragment containing the full length of human GM2A gene was obtained. The obtained PCR fragment was inserted into pET24a vector (catalogue No. 69749-3, Novagen), whereby human GM2A/pET24a plasmid was prepared. Next, after human GM2A/pET24a was added to Escherichia coli BL21 (DE3) pLysS (catalogue No. 69451-4, Novagen) to transform the Escherichia coli, the obtained Escherichia coli was cultured on shaking in an LB medium containing 50 µg/ml kanamycin at 37°C until OD₆₀₀ = about 0.5. Then, after Isopropyl-β-thiogalactopyranoside (catalogue No. 9030, Takara Bio Inc.) was added to the obtained culture so that the final concentration could be 0.5 mM, the culture was further cultured on shaking at 18°C for 3 hours. Bacterial cells were collected by centrifuging the obtained culture, and the collected bacterial cells were sonicated. Then, the obtained homogenate was centrifuged at 11,000 rpm for 30 minutes. After Ni-NTA agarose (catalogueNo. 1018244, Qiagen) was added to the obtained supernatant and left at 4°C for 2 hours, the obtained left object was washed 5 times. From the obtained washed object, an elution fraction of a protein was obtained by elution of a protein with 250 mM imidazole. The obtained elution fraction was purified by anion exchange chromatography HiTrapQ HP, whereby human GM2A protein was prepared.

A test on signaling inhibition by GM2A protein was conducted as follows.

After adding human GM2A protein to cells of differentiated mouse skeletal muscle cell line C2C12 and culturing the cells for 24 hours, the liquid medium was removed from the obtained culture and then exchanged for a D-MEM not containing glucose and serum (catalogue No. 11966, Gibco), and the culture was further cultured for 2 hours. Next, 1 µg/ml insulin (catalogue No. I-5500, Sigma-Aldrich Co.) was added to the obtained culture, and the culture was cultured for 10 minutes. Then, the obtained cell was washed with 10 mM Tris-HCl (pH 7.5). Thereafter, a cell extract was obtained from the obtained cells in a manner similar to the method used in the above-mentioned example. In the same manner, human GM2A protein was added to cells of undifferentiated mouse skeletal muscle cell line C2C12 and the culture was cultured for 24 hours. Thereafter, the liquid medium was removed from the obtained culture and then exchanged for a D-MEM not containing serum, and the culture was further cultured for 2 hours. Then, after 0.1 µg/ml leptin (catalogue No. 450-31, Peprotech Inc.) was added to the obtained culture and the culture was cultured for 30 minutes, a cell extract was prepared from the obtained cells in a manner similar to that described above. In addition, human GM2A protein was added to cells of rat pheochromocytoma cell line PC12, and the cells were cultured for 24 hours. Thereafter, the liquid medium was removed from the obtained culture and then exchanged for a D-MEM not containing serum, and the culture was further cultured for 1 hour. Next, 0.1 µg/ml NGF (catalogue No. 147-04641, Wako Pure Chemical Industries, Ltd.) was added to the obtained culture and the culture was cultured for 10 minutes. Thereafter, a cell extract was prepared from the obtained cells in a manner similar to that described above.

According to the method described in Example 3, after blotting the cell extract to a PVDF membrane, blotting was carried out using anti-phosphorylated Akt antibody (catalogue No. 9271, Cell Signaling Technology) and anti-Akt antibody (catalogue No. 9272, Cell Signaling Technology) for insulin, anti-phosphorylated AMPK α antibody (catalogue No. 2531, Cell Signaling Technology) and anti-AMPK α antibody (catalogue No.2603,Cell Signaling Technology) for leptin, and anti-phosphorylated Akt antibody and anti-Akt antibody for NGF. Next, the luminescence intensity of each band was measured, and relative amount of phosphorylated protein relative to nonphosphorylated protein was calculated. The results are shown in Table 5.

**Table 5**

| Insulin | GM2AP (µg/ml) | Phosphorylated Akt/Akt |
|---|---|---|
| - | 0 | 1.0 |
| + | 0 | 2.1 |
| + | 2 | 1.8 |
| + | 4 | 1.2 |

| LepLin | GM2AP (µg/ml) | Phosphorylated Akt/Akt |
|---|---|---|
| - | 0 | 1.0 |
| + | 0 | 1.9 |
| + | 2 | 1.0 |
| + | 4 | 0.9 |

| NGF | GM2AP (µg/ml) | Phosphorylated Akt/Akt |
|---|---|---|
| - | 0 | 1.0 |
| + | 0 | 1.8 |
| + | 2 | 1.5 |
| + | 4 | 1.4 |

As is clear from the above-mentioned test results, GM2A protein inhibited intracellular signaling of insulin, leptin and NGF in a concentration-dependent manner.

### Example 6 (Quantitative Determination Method of GM2A Protein) (1) (Preparation of Rabbit Anti-Human GM2A Antibody)

From an antiserum obtained by immunizing a rabbit with a peptide consisting of the amino acid sequence of SEQ ID NO: 3 or SEQ ID NO: 4, anti-human GM2A antibody I or anti-human GM2A antibody II was obtained respectively, as a rabbit anti-human GM2A polyclonal antibody, using an antigen column. In order to check reactivity of the obtained anti-human GM2A antibody and GM2A protein, a gel obtained by electrophoresing human, mouse and rat GM2A was blotted with each of the above-mentioned antibodies at the rate of 1 µg/ml. As a result, it was checked that anti-human GM2A antibody I and anti-human GM2A antibody II react with all of human, mouse and rat GM2A proteins.
SEQ ID NO: 3: CysAspGluGlyLysAspProAlaValIleArgSerLeu SEQID NO: 4: CysProPheLysGluGlyThrTyrSerLeuProLysSerGluPheValVal

### (2) Biotin Labeling of Anti-Human GM2A Antibody II

Using Peroxidase Labeling Kit-NH₂ (DOJINDO LABORATORIES), 200 µg of anti-human GM2A antibody II was labeled with HRP according to the protocol of the kit (hereinafter referred to as anti-human GM2A antibody II-HRP).

### (3) ELISA

Anti-human GM2A antibody I was diluted to 100-foldwitha coating buffer (25 mM NaHCO₃, 25 mM Na₂CO₃), to prepare an antibody solution of 10 µg/ml. The obtained antibody solution was added to a 96-well plate at the rate of 100 µl per well, and left at room temperature for 1 hour. After removing the antibody solution by aspiration, the plate was washed using a wash buffer (50 mM Tris-HCl (pH 8.0), 0.14MNaCl) at the rate of 200 µl per well, and the washing manipulation was repeated 3 times. Next, 200 µl of a wash buffer containing 1% BSA (hereinafter referred to as a blocking buffer) was added to the obtained washed object, and left at room temperature for 2 hours followed by washing with a wash buffer 3 times. Next, 100 µl of the specimen was added to the obtained washed object, and left at room temperature for 1 hour followed by washing with a wash buffer 5 times. Anti-human GM2A antibody II-HRP was diluted 1000-fold with a blocking buffer, to prepare a 1 µg/ml antibody solution for detection. The obtained antibody solution for detection was added at the rate of 100 µl per well, and left at room temperature for 1 hour. After washing the obtained left object with a wash buf fer 5 times, 100 µl of TMB peroxidase substrate solution (catalogue No. 50-76-01, KPL) was added thereto. After leaving the obtained mixture for about 15 minutes, 100 µl of 2 M sulfuric acid was added thereto to stop the reaction. Next, absorbance at 450 nm of each well containing the obtained reactant was measured. Using human GM2A protein as a standard, linearity was observed within the range of 1 ng/ml to 1 µg/ml.

### Example 7 (Quantitative Determination of GM2A Protein in Biological Sample)

### (1) Normal Rat Visceral Adipocyte Culture Supernatant

A normal rat visceral adipocyte was cultured by a method similar to the method used in Example 1, and a culture supernatant of each of Days 3, 5, 7 and 8 was collected. Next, the amount of GM2A protein in the culture supernatant was quantitatively determined by a method similar to the method used in Example 6(3). The results are shown in Table 6.

**Table 6**

| | GM2A Protein (µg/ml) | | | |
|---|---|---|---|---|
| | Day 3 | Day 5 | Day 7 | Day 8 |
| Visceral Adipocyte | 0.41 | 0.42 | 0.52 | 0.68 |

The GM2A protein in the culture supernatant increased in accordance with increase in the number of days of culture (that is, accumulation of lipid droplet).

### (2) Human Serum

Thirteen (13) specimens of nonobese serum and 7 specimens of obese patient serum were purchased from ILSbio. After diluting each purchased serum specimen 20- to 100-fold, the amount of GM2A protein in human serum was quantitatively determined by a method similar to the method used in Example 6(3). The results are shown in Table 7.

**Table 7**

| | GM2A Protein (µg/ml) |
|---|---|
| Nonobese | 7.0 |
| Obese | 10.5 |

In obese patients, was shown significantly higher value of the amount of GM2A protein in the serum relative to that of nonobese patients (p < 0.05).

Example 8 (Inhibitory Action by GM2A Protein on Glucose Uptake)

Mouse adipocyte cell line 3T3-L1 was purchased from Health Science Research Resources Bank. Cells of the purchased mouse adipocyte cell line 3T2-L1 were cultured in a D-MEM containing 10% fetal bovine serum (hereinafter referred to as D-MEM (10)) at 37°C under an atmosphere of 5% CO₂. The cultured cells were plated on a 6-swell plate. Two days after becoming confluent, the culture solution was exchanged for a pre-differentiation culture solution (D-MEM (10) containing 1 µg/ml insulin, 0.5 mM 3-isobutyl-1-methylxanthine, and 1 µM dexamethasone), and the culture was further cultured for another 4 days. Next, after the pre-differentiation culture solution was exchanged for a post-differentiation culture solution (D-MEM (10) containing 1 µg/ml insulin) and the culture was cultured for 3 days, the culture solution was exchanged for a D-MEM (10) containing mouse GM2A protein, and the culture was further cultured for another day. On the following day, after removing the culture solution from the obtained culture, the culture solution was exchanged for a post-differentiation culture solution containing mouse GM2A protein or a D-MEM (10), and the culture was further cultured for 4 hours. Then, after washing the obtained cells with an HRPH buffer (20 mM HEPES-OH (pH 7.4), 5 mM KH₂PO₄, 1 mM MgSO₄, 1 mM CaCl₂, 136 mM NaCl, 4.7 mM KCl, 0.1% bovine serum albumin) twice, an HRPH buffer containing 1 mM 2-deoxyglucose was added thereto, and the mixture was cultured for 30 minutes. Next, after washing the obtained culture with an HRPH buffer twice, cells were collected using a scraper. Then, after adding 0.1 M NaOH to a precipitate obtained by centrifuging the collected object, the cells were freeze-thawed by vortexing the mixture. After keeping the freeze-thawed cells (cell solution) at 85°C for 30 minutes, a specimen was prepared by adding 0.1 MHCl thereto to neutralize the solution. Here, the protein concentration of the specimen was measured using BCA Protein Assay Reagent (catalogue No. 23228, Pierce) using bovine serum albumin as a standard protein.

The amount of 2-deoxyglucose in the specimen was quantitatively determined according to the following method. To a plate for fluorescence measurement, were added 100 µl of an assay buffer (50 mM triethanolamine (pH 8.1), 50 mM KCl, 0.5 mM MgCl₂, 0.02% bovine serumalbumin, 670 µM ATP, 0.12 µM NADP⁺, 25 µM resazurin, 5.5 units/ml hexokinase, 16 units/ml G6PDH, 1 units/ml diaphorase) and 100 µl of the specimen. After keeping the obtained mixture at 37°C for 90 minutes, the fluorescence intensity of the obtained mixture was measured (excitation wavelength: 530 nm, detection wavelength: 595 nm). A calibration curve was created using 2-deoxyglucose of 500 µM, 250 µM, 125 µM and 0 µM (purified water) as a standard, and quantitative determination was carried out on the basis of the calibration curve. The amount of 2-deoxyglucose incorporated into a cell per the amount of protein of the specimen was calculated as a relative value. The results are shown in Table 8.

**Table 8**

| Insulin | GM2A Protein (µg/ml) | Amount of 2-Deaxyglucose |
|---|---|---|
| - | 0 | 100 |
| + | 0 | 215 |
| + | 2 | 184 |
| + | 10 | 156 |

GM2A protein inhibited an enhancing action by insulin on intracellular incorporation of 2-deoxyglucose in a concentration-dependent manner.

### Example 9 (Enhancing Action by Anti-GM2A Antibody and Ligand on Glucose Uptake)

After adding anti-human GM2A antibody II, which is an antibody to a ligand-binding domain of human GM2A, and a ganglioside GM2 (catalogue No. G8397, Sigma-Aldrich Co.), which is a ligand of human GM2A, to cells of differentiated mouse adipocyte cell line 3T3-L1 (secreting GM2A protein into a culture solution), the amount of 2-deoxyglucose in the cells was measured. After culturing the cells in a post-differentiation culture solution for 3 days by a method similar to the method used in Example 8, the culture solution was exchanged for a post-differentiation culture solution containing anti-human GM2A antibody II or rabbit IgG (control antibody), or a ganglioside GM2 or DMSO, and cultured for 1 day. On the following day, after removing the culture solution from the obtained culture and then washing the cells with an HRPH buffer twice, an HRPH buffer containing 1 mM 2-deoxyglucose was added thereto, and the mixture was cultured for 30 minutes. The amount of 2-deoxyglucose in the specimen was measured by a method similar to the method used in Example 8. The amount of 2-deoxyglucose incorporated into the cells was calculated as a relative value. The results are shown in Table 9.

**Table 9**

| Antibody or Ligand | Amount of 2-Deoxyglucose |
|---|---|
| Control Antibody 10 µg/ml | 100 |
| Anti-Human GM2A Antibody II 10 µg/ml | 125 |
| DMSO | 100 |
| Ganglioside GM2 1 µM | 120 |

Anti-human GM2A antibody II and a ganglioside GM2 enhanced intracellular incorporation of 2-deoxyglucose.

### INDUSTRIAL APPLICABILITY

The method of the present invention can be utilized for early detection of a microdomain disease and monitoring of the disease state and so on, and the animal of the present invention can be utilized for drug development and so on of a prophylactic or therapeutic agent or the like for microdomain disease. In addition, an anti-GM2A antibody and a GM2A-inhibitory active compound can be utilized as a prophylactic or therapeutic agent for a microdomain disease.

### Free Text in Sequence Listing

### SEQ ID NO:1

Primer

### SEQ ID NO:2

Primer

### SEQ ID NO:3

Antigenic peptide

### SEQ ID NO:4

Antigenic peptide

## Claims

1. A method for detecting a microdomain disease in a test animal comprising a step of measuring the amount or activity of GM2A in a biological sample obtained from the test animal and a step of comparing the measured amount or activity of GM2A with a threshold.

2. A method for monitoring change in a disease state of a microdomain disease comprising a step of measuring the amount or activity of GM2A in a biological sample obtained from a test animal and a step of comparing the measured amount or activity of GM2A with the amount or activity of GM2A measured for the test animal at differing time points.

3. The method according to claim 1 or 2, wherein the biological sample is blood, lymph, a tissue or a cell.

4. The method according to claim 1 or 2, wherein the measurement of the amount or activity of GM2A is carried out by enzyme immunoassay, radioimmunoassay, fluoroimmunoassay, ELISA, immunohistochemical staining, immunoprecipitation, Westernblotting, Northernblotting or RT-PCR.

5. Use of GM2A as a biomarker for a microdomain disease.

6. The use according to claim 5, wherein the microdomain disease is obesity, hyperlipidemia, hypertension, arteriosclerosis, diabetes mellitus or a complication thereof, cancer, central nervous system disorder, endometriosis, osterioporosis or autoimmune disease.

7. A kit for diagnosis of a microdomain disease which comprises a reagent for measuring the amount or activity of GM2A.

8. The kit for diagnosis according to claim 7, wherein the reagent for measuring the amount or activity of GM2A is an anti-GM2A antibody, or a ganglioside labeled with a radioisotope or a fluorescent dye.

9. A nonhuman transgenic animal into which a gene modified so that the expression level of GM2A can increase has been introduced.

10. The nonhuman transgenic animal according to claim 9, wherein the expression level of GM2A is increased in an adipocyte.

11. A nonhuman animal to which GM2A protein has been administered to artificially increase the amount or activity of GM2A in blood.

12. The nonhuman animal according to any one of claim 9 to 11, wherein a symptom of a microdomain disease is exhibited.

13. A prophylactic or therapeutic agent for a microdomain disease which comprises an anti-GM2A antibody as an active ingredient.

14. A prophylactic or therapeutic agent for a microdomain disease which comprises a GM2A-inhibitory active compound as an active ingredient.


SEQUENCE LISTING

<110>  Sumitomo Chemical Co., Ltd.

<120>  Biomarker for microdomain disease

<130>  S21753W001

<150>  JP 2008-246846
<151>  2008-09-25

<160>  4

<170>  PatentIn version 3.4

<210>  1
<211>  18
<212>  DNA
<213>  Artificial

<220>
<223>  Primer

<400>  1
gtgctgggct tgctgttc                                                    18

<210>  2
<211>  20
<212>  DNA
<213>  Artificial

<220>

<223>  Primer

<400>  2
gatgctctgg atgcggtagt                                                    20

<210>  3
<211>  13
<212>  PRT
<213>  Artificial

<220>
<223>  Antigenic peptide

<400>  3
Cys Asp Glu Gly Lys Asp Pro Ala Val Ile Arg Ser Leu
1               5                   10

<210>  4
<211>  17
<212>  PRT
<213>  Artificial

<220>
<223>  Antigenic peptide

<400>  4
Cys Pro Phe Lys Glu Gly Thr Tyr Ser Leu Pro Lys Ser Glu Phe Val
1               5                   10                  15
Val